Europäisches Patentamt

European Patent Office

Office européen des brevets

(11) Publication number : **0 521 799 A1**

## (12) EUROPEAN PATENT APPLICATION

(21) Application number : **92401927.6**

(22) Date of filing : **03.07.92**

(51) Int. Cl.⁵ : **A61K 9/107**

(30) Priority : **05.07.91 IL 98747**

(43) Date of publication of application :
**07.01.93 Bulletin 93/01**

(84) Designated Contracting States :
**AT BE CH DE DK ES FR GB GR IT LI LU MC NL PT SE**

(71) Applicant : **YISSUM RESEARCH DEVELOPMENT COMPANY OF THE HEBREW UNIVERSITY OF JERUSALEM
46 Jabotinsky Street
Jerusalem, 92 182 (IL)**

(72) Inventor : **Benita, Simon
33/3 Haarazim Street, Mevasseret Zion
IL-90805 Jerusalem (IL)**
Inventor : **Muchtar, Shaul
20 Ha'elah Street
Reut (IL)**

(74) Representative : **Hubert, Philippe et al
Cabinet Beau de Loménie 55, rue
d'Amsterdam
F-75008 Paris (FR)**

(54) **Ophthalmic compositions.**

(57)   Water insoluble drugs are administered to the eye within a drug delivery system which is an oil-in-water type emulsion which comprises oil, phospholipids and an amphoteric surfactant.

STABILITY - MEAN DROPLET SIZE EVALUATION
AT FIVE STORAGE TEMPERATURES
EMULSION - M

(a)

Legend: 50 C, 37 C, 30 C, 25 C, 4 C

STABILITY - POLYDISPERSITY OF DROPLET SIZE
AT FIVE STORAGE TEMPERATURES
EMULSION - M

(b)

Fig. 8

Jouve, 18, rue Saint-Denis, 75001 PARIS

## FIELD OF THE INVENTION

The present invention is generally in the field of drug delivery and provides an ophthalmic drug delivery vehicle of the oil-in-water type emulsion. The present invention further provides an ophthalmic composition comprising a hydrophobic, amphophilic or lipophilic drug and the above vehicle.

As used herein, the term "hydrophobic drug" means a drug which is sparingly soluble in water; the term "amphophilic drug" means a drug which is soluble to a noticeable degree in both oil and water, e.g. has an oil-in-water partition coefficient of about 1.0; and the term "lipophilic drug" means a drug which has an oil solubility considerably exceeding its water solubility.

## BACKGROUND OF THE INVENTION AND PRIOR ART

In the following description reference will at times be made to various prior art documents by indicating in brackets their number from a list of references to be found at the end of the description before the claims.

Aqueous solutions are by far the most common vehicles for ophthalmic drugs used to date. However, such vehicles have a serious drawback in that the ocular bioavailability of drugs administered thereby is generally very poor due to rapid drainage and tear turnover (1). An instilled dose of ophthalmic solution, which is typically in the range of 50-100µl far exceeds the normal lacrimal volume of 7-10µl and thus the portion of the instilled dose that is not eliminated by spillage from the palpebral fissure is quickly drained. Furthermore, often after instillment of an ophthalmic solution, lacrimation and physiological tear turnover, which in humans is about 16% per minute under normal conditions, increases, resulting in rapid dilution of the applied drug that has not been spilled or drained. In consequence to all the above, the contact time with the absorbing surfaces of the eye (cornea and sclera) of drugs which are applied to the eye within liquid aqueous compositions is reduced to less than 2 minutes.

Another drawback of aqueous vehicles is that many drugs which may potentially be used in eye therapy are hydrophobic and their delivery into the eye by such vehicles is not possible. While such drugs may potentially be administered to the eye by various organic, e.g. alcoholic, solvents, the use of such solvents usually causes irritation and inflammatory reactions (2).

Attempts have been made to develop various delivery vehicles in which the drug resistance time in the eye is increased. The most direct approach for achieving this goal is by an increase in the vehicle's viscosity and various viscous vehicles such as hydrogels or ointments have been developed, some of which also enable delivery into the eye of hydrophobic drugs. Additionally, many attempts to use various non-conventional carriers such as liposomes, micellar solutions and nano-particles as vehicles of ophthalmic drugs, were also made, but while the use of such delivery system gave rise to a limited success in prolonging the residence time of drugs in the eye and hence some enhancement of the ocular bioavailability, such carriers had also various side effects (2, 3, 4).

It has already been suggested that emulsions may be used as vehicles for delivery of drugs to the eye (5,6). However, to date no successful ophthalmic compositions in the form of oil-in-water emulsions are available.

## OBJECTS OF THE PRESENT INVENTION

It is an object of the invention to provide an ophthalmic drug delivery vehicle for hydrophobic, amphophilic or lipophilic drugs of the oil-in-water type emulsion.

It is also an object of the invention to provide an ophthalmic composition comprising hydrophobic, amphophilic and lipophilic drugs and the above vehicles.

## SUMMARY OF THE INVENTION

In accordance with the invention it has been found that the above object may be achieved by an oil-in-water type emulsion comprising an oily carrier, phospholipids and an amphoteric surfactant.

The term "amphoteric surfactant" as used herein means a surface active agent having both acidic and basic characteristics, i.e. its hydrophilic portion is cationic in acidic solution and anionic in basic solution.

The invention thus provides an ophthalmic drug delivery vehicle of hydrophobic, amphophilic or lipophilic drugs of the oil-in-water type emulsion, comprising about 0.5-50% oil, about 0.5-10% phospholipids and about 0.05-5% of an amphoteric surfactant.

The present invention further provides a pharmaceutical ophthalmic composition comprising an effective amount of a hydrophobic, amphophilic or a lipophilic drug and said drug delivery vehicle.

2

The composition of the present invention is suitable for use in human and veterinary medicine.

All indication of (%) both above and below are by weight (weight of ingredients/weight of the entire composition). All indicated concentrations above and below should be understood as standing each by itself and not accumulative. It should however be appreciated by the artisan that there is some dependency of the concentrations of one ingredient to another, e.g. a high concentration of the oil will generally require a high concentration of the phospholipids and the surfactant.

Preferred ranges of ingredients are as follows: oil - 1-20% for composition intended to be fluid or 30-50% for a viscous composition; phospholipids - 0.2-5%, 0.2-1% being particularly preferred; amphoteric surfactant - 0.2-2%, 0.2-0.5% being particularly preferred.

It was found in accordance with the present invention that the preferred ratio between the concentration of the phospholipids to that of the amphoteric surfactant is about 1:1 to about 4:1, ratio of about 2:1 being particularly preferred.

The present invention further provides a process for preparing an ophthalmic pharmaceutical composition comprising incorporating a hydrophobic, amphophilic or lipophilic drug into an ophthalmic acceptable drug delivery vehicle, characterized in that said vehicle is an oil-in-water type emulsion comprising about 0.5-50% oil, about 0.5-10% phospholipid and about 0.2-10% of an amphoteric surfactant.

The invention still further provides a method of treatment of the eye, comprising administering a hydrophobic, amphophilic or a lipophilic drug suitable for the treatment of ocular diseases or disorders, characterized in that said drug is delivered into the eye in a delivery system being an oil-in-water type emulsion comprising about 0.5-50% oil, about 0.5-10% phospholipids and about 0.2-10% of an amphoteric surfactant.

The method is applicable in both human and non-human animals.

## DETAILED DESCRIPTION OF THE INVENTION

In accordance with the present invention, hydrophobic drugs are administered to the eye in a vehicle being an oil-in-water type emulsion comprising oil, phospholipids and an amphoteric surfactant in the concentrations specified above. In ophthalmic preparations, care must be made to avoid the use of ingredients which may potentially initate the eye. Accordingly, all the ingredients used in the vehicle and compositions of the present invention, should preferably be highly purified to avoid contamination by possible irritating substances.

The oil in the emulsion may be a vegetable oil, a mineral oil, medium chain triglyceride (MCT) oil, (i.e. a triglyceride oil in which the carbohydrate chain has 8-12 carbon atoms), oily fatty acids, isopropyl myristate, oily fatty alcohols, esters of sorbitol and fatty acids, oily sucrose esters or a combination of different types of oils mentioned above and in general any oily substances which is physiologically tolerable when administered into the eye.

Examples of MCT oil are TCM™ (Societe des Oleagineux, France) which is a mixture of triglycerides wherein about 95% of the fatty acid chains have either 8 or 10 carbon atoms, and MIGLYOL 812™ (Dynamit Nobel, Sweden) which is a mixed triester of glycerine and of caprilic and capric acids.

Examples of vegetable oil are soya bean oil, cotton seed oil, olive oil and sesame oil.

Examples of oily fatty acids are oleic acid and linoleic acid. An example of a fatty alcohol is oleyl alcohol. Examples of esters of sorbitol and fatty acids are sorbitan monoleate and sorbitan monooleate. Examples of oily sucrose esters are sucrose mono-di-or tri-palmitate.

Examples of phospholipids are lethicin, EPIKURON 120™ (Lucas Meyer, Germany) which is a mixture of about 70% phosphatidylcholine, about 12% phosphatidylethanol-amine and about 15% other phospholipids; OVOTHIN 160™ (Lucas Meyer, Germany) which is a mixture comprising about 60% phosphatidylcholine, about 18% phosphatidylethanolamine and about 12% other phospholipids; a purified phospholipid mixture; LIPOID E-80™ (Lipoid, Germany), which is a phospholipid mixture comprising about 80% phosphatidylcholine, about 8% phosphatidylethanolamine, about 3.6% non-polar lipids, and about 2% sphingomyelin.

Hitherto, the use of emulsions in ophthalmic preparations has been limited to a large extent by the fact that the surfactants generally used in emulsions are highly irritating to the eye. For example, although the emulsion preparation disclosed in EP-A-391,369 (5) may also in principle be used as an ophthalmic preparation, as also stated therein, such use will be considerably limited by the irritating effect of the non-ionic and in particularly the ionic surfactant used there. Against this, the amphoteric surface active agents used in accordance with the present invention have generally no irritating effect on the eye. A particularly preferred amphoteric surface active agent in accordance with the present invention is an imidazoline based amphoteric such as MIRANOL C2M™ and MIRANOL MHT™ (both - Miranol Inc., Dayton, N.J., U.S.A.) and various other products sold under the tradename MIRANOL™. In addition to MIRANOL™, various other amphoteric-foaming agents such as those used in various baby shampoos, may also be used in compositions in accordance with the present invention.

Health regulations in various countries generally require that ophthalmic preparations shall comprise a preservative. Many preservatives customarily used in ophthalmic preparations cannot be used in compositions of the present invention, since various such preservatives can interact with the surfactant to form a complex whereby their bacteriocidic activity is considerably reduced; furthermore, many preservatives can be absorbed by droplets in the emulsion, which absorption also reduces their bacteriocidic activity. The preservatives should thus be chosen from among such which do not interact with the surfactant or are not absorbed by the droplets to such an extent in which they will be prevented from protecting the emulsion from microbiological contamination. Examples of such preservatives are thimerosal (e.g. at a concentration of about 0.01-0.2%) and chlorbutol (e.g. at a concentration of about 0.01-0.2%). Particularly preferred is a composition comprising a combination of both of these preservatives, which composition was found to have improved properties.

The composition of the present invention may comprise various drugs such as, for example, drugs used for the treatment of glaucoma, anti-inflammatory drugs, antibiotic and anti-cancer drugs, anti-fungal drugs and anti-viral drugs.

Examples of anti-glaucoma drugs are $\beta$-blockers such as timolol-base, betaxalol, athenolol, livobanolol, epinenephrin, dipivalyl, oxonolol, and other drugs such as acetazilumide-base and methazalomide.

Examples of anti-inflammatory drugs are steroidal drugs such as cortisone and dexamethasone and non-steroid anti-inflammatory drugs (NSAID) such as piroxicam, indomethacine, naproxen, phenylbutazone, ibuprofen and diclofenac-acid.

An example of an antibiotic and anti-cancer drugs is chloramphenicol; examples of anti-fungal drugs are nystatin and myconazole; and an example of an anti-viral drug is Acyclovir. Examples of anti-allergic drugs are pheniramide derivatives and sodium chromoglycate.

It is generally preferred that the diameter of the oily droplets in the emulsion be below about 1$\mu$m, desirably below about 0.5$\mu$m and preferably below about 0.2$\mu$m. Small droplets are preferred since such emulsions have a higher degree of stability particularly in steam autoclaving. Furthermore small droplets enable sterilization by filtration.

The composition of the present invention may also comprise various osmotic pressure regulators such as mannitol or glycerine as well as an anti-oxidant such as $\alpha$-tocopherol.

It was surprisingly found that, for reasons which have not yet been elucidated, with the compositions of the present invention, the drug has at times a prolonged pharmacological effect in the eye: the effect of some drugs administered by the composition of the present invention was found to last up to about 36 hours. Such a long duration of pharmacological activity in the eye, compared with a short duration of pharmacological activity of drugs administered by a conventional liquid composition, is of great advantage for the treatment of a large number of acute and chronic eye diseases.

The drug delivery vehicle and the composition of the invention are capable of withstanding sterilization in an autoclave essentially without a loss in their properties, i.e. drug content (provided of course that the drug is not heat sensitive) or droplet size. This is an important feature seeing that sterilization by autoclave is the most convenient, and thus a generally preferred method of sterilization of liquid pharmaceutical compositions comprising a heat resistant drug.

The drug delivery vehicle and the compositions of the invention maintain their properties even at acidic pH, e.g. down to pH 2-3. This is also a very important feature since, for one, many drugs require an acidic environment to exert their activity and furthermore during prolonged storage some of the triglycerides may break down, giving rise to an increase in concentration of fatty acids and hence a decrease in pH.

The composition of the present invention may be prepared in a number of ways. By one way of preparation an aqueous solution and an oily solution are first separately prepared, the aqueous solution comprising the amphoteric surfactant and if present, also the osmotic pressure regulator and the preservative and the oily solution comprising the oil, the phospholipid, the hydrophobic drug and if present, also the antioxidant.

The aqueous solution and the oily solution are then mixed with one another, preferably after each has been separately heated. However, the so-obtained mixture does not yet consist of sufficiently small droplets, the size of which (obtained after mixing. e.g. with a magnetic stirrer) is about 10$\mu$m. The droplet size may then be decreased by the use of emulsification equipment, such as ULTRA TURRAX™ (Jenkle and Kunkel, Stauffen, Germany), which yields droplets having an average diameter of about 1.1$\mu$m, or of a high shear mixer, e.g. POLYTRON™ (Kinematica, Lucerne, Switzerland) which yields droplets having an average diameter of about 0.6$\mu$m.

Smaller droplets may be obtained when utilizing a two stage pressure homogenizer, in which the crude dispersion is forced under high pressure through the annular space between a spring loaded valve and then through the valve seat, the second stage being in tandem with the first so that the emulsion is subjected to two very rapid dispersion processes. Example of such an apparatus is the GAULIN™ homogenizer (A.P.V. GAULIN, Hilversum, The Netherlands or A.P.V. RANNIE, Albertsland, Denmark). After homogenization in such an apparatus, the emulsion droplets have an average diameter of less than 0.3 $\mu$m, with a high degree of uniformity in the droplets size. Even smaller droplets may be obtained when the emulsification process combines the use of both a polytron-type high shear mixer followed by homogenization. The droplets size which are obtained in such a combination have an average diameter of about 0.1-0.15μm.

In the following, the present invention will be illustrated in more detail with reference to several non-limiting embodiments.

## BRIEF DESCRIPTION OF THE DRAWINGS

In the following description reference will at times be made to the annexed drawings in which:

**Fig. 1** shows the content of the drug $\Delta^8$-THC in emulsion "M" (see below) at various storage times and temperatures;

**Fig. 2** shows the content of $\Delta^8$-THC in emulsion "C" (see below) at various storage times and temperatures;

**Fig. 3** shows the effect of the phospholipid concentration on the mean droplet size and the polydispersity;

**Fig. 4** shows the effect of the concentration of the amphoteric surfactant on the mean droplet size;

**Fig. 5** shows the effect of the oil concentration on the mean droplet size and the polydispersity;

**Fig. 6** shows the effect of pH on droplet size (**a**) and polydispersity (**b**) in an emulsion comprising either a non-ionic surfactant or an amphoteric surfactant;

**Fig. 7** shows the effect of divalent cations' concentration on the stability of emulsion containing MIRANOL as compared to an emulsion containing PLURONIC F-68.

**Fig. 8** shows the mean droplet size (**a**) and the polydispersity (**b**) of emulsion M at various storage times;

**Fig. 9** shows the mean droplet size (**a**) and the polydispersity (**b**) of emulsion C at various storage times;

**Fig. 10** shows the mean droplet size (**a**) and polydispersity (**b**) of emulsions containing HU-211 at various storage times and temperatures;

**Fig. 11** shows the HU-211 concentration in emulsions following storage for various periods of times at various temperatures;

**Fig. 12** shows the zeta-potential of emulsion M following storage for various periods of time at various temperatures;

**Fig. 13** shows the IOP measured at various times, starting at 8am on one day and at various intervals thereafter. Both the basal IOP and the IOP after treatment with HU-211 are shown. (**A**) are results obtained from one group of animals (referred to herein as "Group I") and (B) are results obtained from another group of animals (referred to herein as "Group II"). The two groups of animals received exactly the same treatment;

**Fig. 14** shows the drop of IOP following administration of HU-211 as compared to basal levels (**A** -Group I, **B** -Group II);

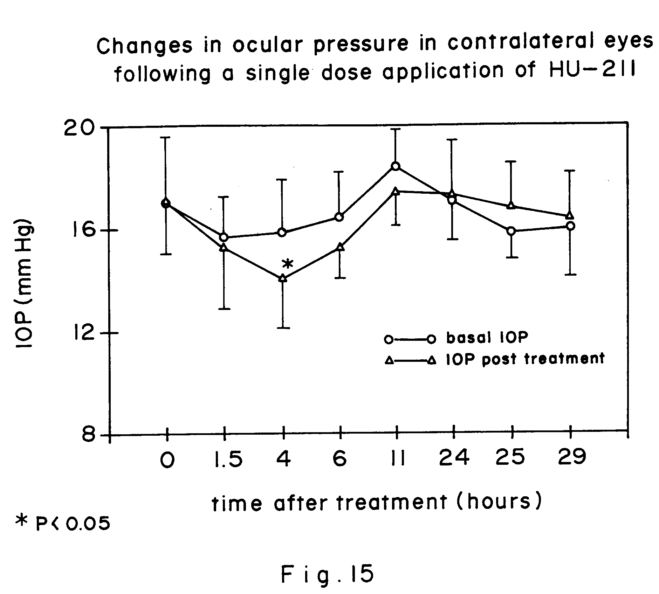

**Fig. 15** shows the basal IOP and that after administration of HU-211 in the contralateral untreated eye;

**Fig. 16** shows the effect of $\Delta^8$-THC on intraocular pressure (IOP) in hypertensive rabbits, administered either in a conventional solution or by means of a composition of the invention; and

**Fig. 17** is a similar experiment to that in Fig. 14 using normo-tensive rabbits;

## EMULSION PREPARATION

### Ingredients

MCT: Societe Industrielle des Oleagnieux, St. Lourent, Blangy, France.

Lipoid E -80: Lipoid, Ludwigshafen, Germany.

Miranol MHT solution (34.5% solid) and Miranol C2M solution (50% solid): Miranol Inc., Dayton, N.J., U.S.A.

$\alpha$-Tocopherol and glycerine: Sygma, St. Louis, MO, USA, in conformity with USP specifications.

$\Delta^8$-THC and HU-211: obtained from Professor Meshulam, Hadassah Medical School, The Hebrew University of Jerusalem, Jerusalem, Israel (7, 8, 9);

Timolol: Merck, Darmstadt, Germany.

Chlorbutol: Sigma, St. Louis, MD., U.S.A.

Thimerosal: Sigma, St. Louis, MD. U.S.A.

Lipoid E -80 was chosen since it is a highly purified mixture of phospholipids consisting of 82% phosphatidylcholine, 8.5% phosphatidylethanolamine, 2% sphingomyelin and 2% lysophosphatidylcholine. A high degree of purification is important in the prevention of eye irritation.

MIRANOL MHT™ and C2M™ were chosen from the entire range of commercial Miranol preparations since they were known to be the least irritating to the eyes, irrespective of their concentrations (10). MIRANOL MHT™ consists of 34.5% lauroamphodiacetate (CAS number 68797-39-7) solid, 3.4% NaCl and water. MIRANOL C2M™ solution consists of 50% cocoamphodiacetate (CAS number 68647-53-0) solid, 11.5% NaCl and water.

The pH of these two solutions was 9.8 and 8.2 respectively. MIRANOL™ amphoteric surfactants are self-buffered and have a buffering capacity throughout the pH range of 4-10. They have an exceptional electrolyte tolerance and are self-preserved against bacterial attack. At neutral pH, the compounds are zwitterions.

Emulsion Preparation

Aqueous and oily phases were separately prepared. The aqueous phase consisted of water, MIRANOL solution, and glycerine and the oily phase consisted of MCT oil, the antioxidant $\alpha$-tocopherol phospholipids and the drug. The pH of the aqueous phase was adjusted to pH 6.8 and the two phases were filtered (TE and BA filter types, Schull & Schleicher, Dassel, Germany, having a pore size of 0.22μm) and heated separately to 70°C. At this temperature, the two phases were combined and stirred with a magnetic stirrer and the mixture was further heated up to a temperature of 85°C. At this temperature, the coarse emulsion was further mixed with a high-shear mixer, POLYTRON™, for 3 minutes, and then rapidly cooled to below 20°C. After cooling, the emulsion was homogenized by a 2-stage homogenizer (EPV RANNIE, Albertsland, Denmark) for 4 minutes at 8000 psi and then cooled again. After adjusting the pH to 6.8-7.0 where the drugs were HU-211 or $\Delta^8$-THC or 7.2 where the drug was Timolol, the emulsion was filtered through a membrane filter (TE, Schuell & Schleicher having a pore size of 0.45 μm) and transferred to plastic bottles that were sealed under nitrogen atmosphere.

Where the preservatives, thimerosal and/or chlorbutol were included in the emulsion, they were added to the final emulsion, prior to sealing of the plastic bottles. In the case where both preservatives were included in the emulsion, thimerosal which easily dissolves in an aqueous solution was added first and chlorbutol was added only after the dissolution of the former.

In the experiment reported hereinbelow, emulsions containing other preservatives were also prepared, the preservatives incorporated into the emulsions by admixture as known *per se.*

The emulsions were then sterilized by a steam autoclave at 121°C for 15 minutes, which brings about also a dissolution of the chlorbutol when same is present in the emulsion.

It should be pointed out that sterilization was at times achieved also by a double stage membrane filtration, first filtration through a 0.45 μm filter followed by filtration through a 0.22 μm filter (both filters were T.E., Shull and Schleicher). Emulsions which were sterilized by filtration contained chlorbutol, which preservative was dissolved by gentle, controlled heating to 40°C for 15 min. in a sealed bottle to prevent its evaporation during the heating phase.

Example of emulsions prepared by the above method

Emulsions containing the following ingredients were prepared (% w/w):

(a)

| MCT oil | : 4.0%; |
| Lipoid E -80 | : 0.75 % |
| $\alpha$-tocopherol | : 0.02% |
| MIRANOL solution | : 1.5% |
| Glycerine - | : 2.25% |
| Water | : to 100% |

(b)

| MCT oil | : 3.5 % |
| Oleic acid | : 1.5 % |
| Lipoid E-80 | : 0.75% |
| Miranol Solution | : 1.5 % |
| $\alpha$-tocopherol | : 0.02% |
| Thimerosal | : 0.01 % |
| Chlorbutol | : 0.2% |
| Water: | : to 100% |

(c)

| MCT: | : 4.25% |
| Phospholipid: | : 0.75% |
| $\alpha$-Tocopherol: | : 0.02% |
| Miranol Solution | : 1.5% |
| Glycerine: | : 2.25% |
| Water: | : to 100% |

The drugs HU-211, $\Delta^8$-THC or Timolol were added to the emulsion up to their desired concentrations.

The MIRANOL solution was either MIRANOL MHT (35% dry weight concentration) or MIRANOL C2M (50% by weight concentration).

In various experiments, the concentration of either MIRANOL solution, the phospholipids or MCT oil was changed.

## Experimental Results

### Test No. 1: Stability Assessment

Emulsions containing the drug $\Delta^8$-THC were prepared with either a MIRANOL MHT (Emulsion M) or MIRANOL C2M (Emulsion C) and the drug concentration after various storage periods at 5 different temperatures between 4-50°C was determined.

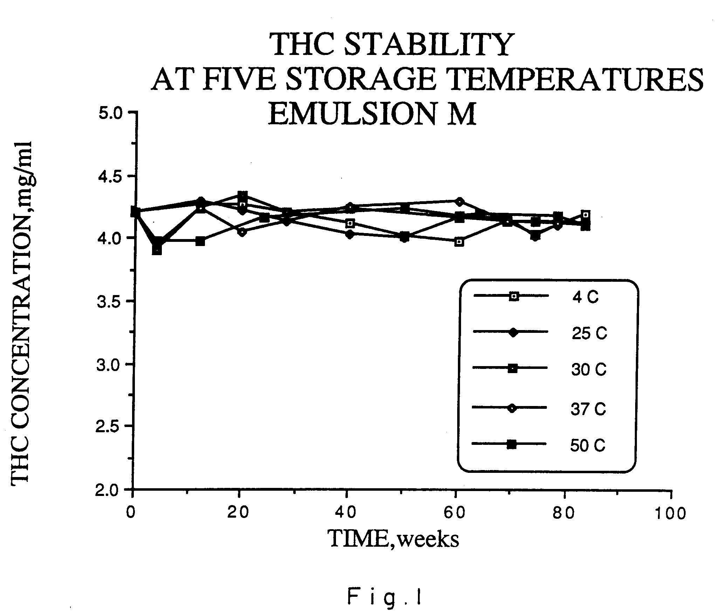

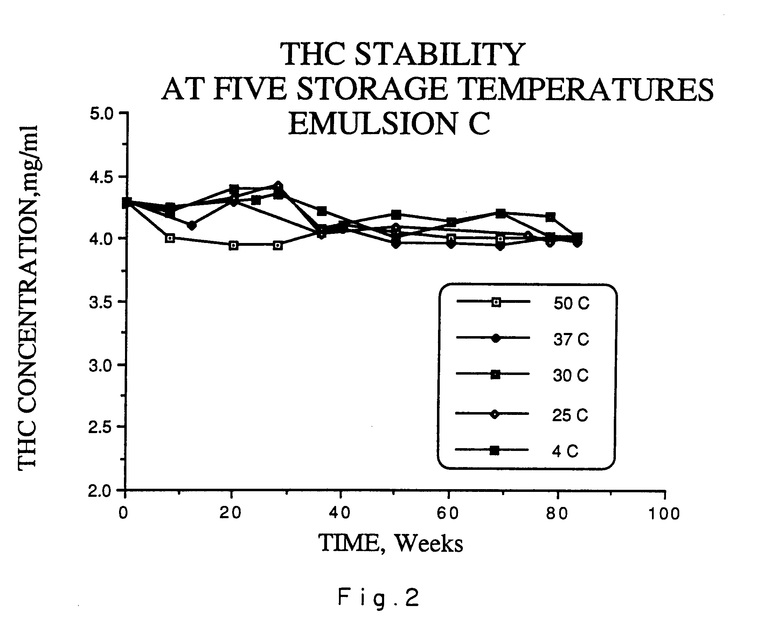

The results of the chemical stability evaluation are shown in Fig. 1 (Emulsion M) and Fig. 2 (Emulsion C). As can be seen from Fig. 1, there was no change in $\Delta^8$-THC concentration irrespective of storage temperatures over 83 weeks storage. As can be seen in Fig. 2, the degradation of the drug in Emulsion C is slightly more pronounced (about 10-15%) than emulsion M in all storage temperatures. Despite contents fluctuation noted over 83 weeks at 37°C and 50°C storage temperature, $\Delta^8$-THC appeared to be stable in Emulsion C at 4°C, 25°C and 30°C.

### Test No. 2

#### Effect of phospholipid concentration on mean droplet size

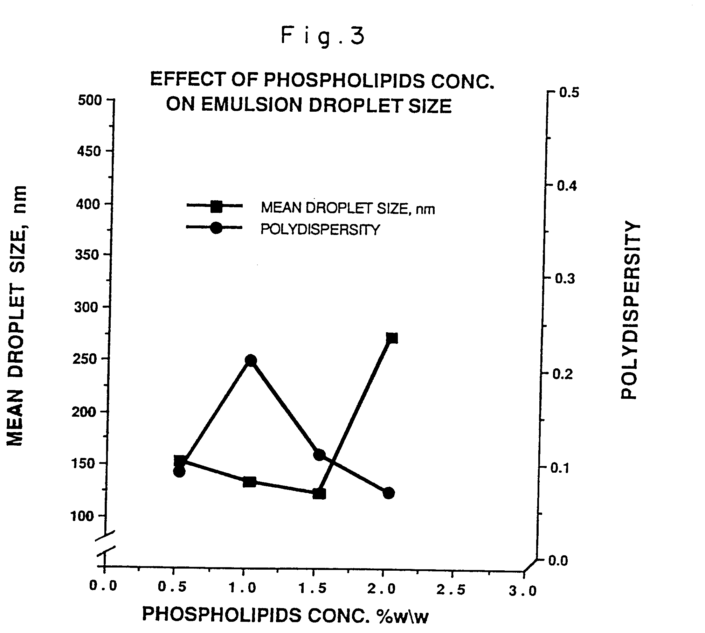

5 emulsions were prepared containing different concentrations of LIPOID E-80 (between 0-2.0%). The mean droplet size and the polydispersity (a measure of the droplets' homogeneity - the smaller the polydispersity, the higher is the homogeneity) was measured using photon correlation spectroscopy (COULTER-4, Version 9.1). As can be seen in Fig. 3, the lowest droplet size was achieved at a concentration of phospholipids of 1.5%. At a concentration of 2%, the droplet size increased probably due to the repulsion of the amphoteric surfactant from the oil-water interface by the high phospholipid concentration. Emulsions with no phospholipids were unstable and phase separation occurred a few minutes after preparation.

### Test No. 3:

#### Effect of MIRANOL concentration on the mean droplet size

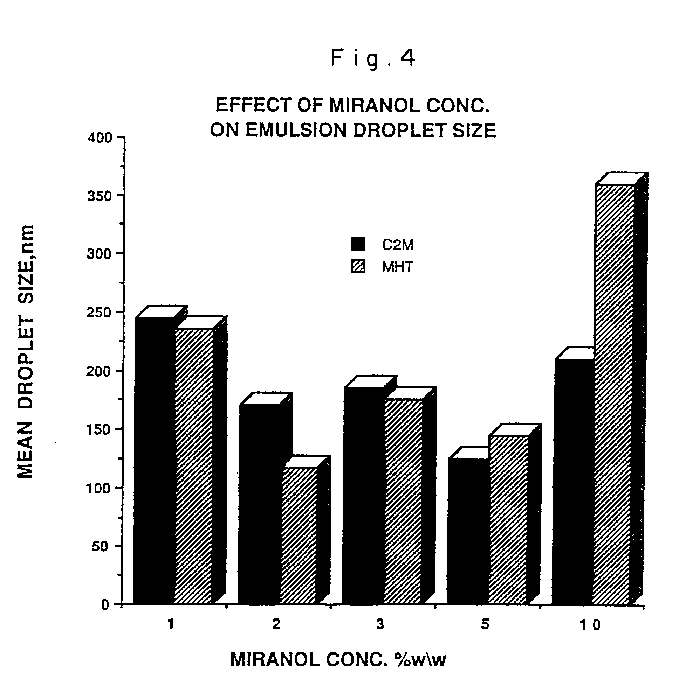

6 emulsions containing different concentrations of MIRANOL MHT and MIRANOL C2M solutions (from 0-10%) were prepared and the mean droplet size was measured. The results are shown in Fig. 4. As can be seen in this figure, the smallest droplet size was achieved at a concentration of the MIRANOL solution of between 2-5% (2% minimum for MIRANOL MHT and 5% minimum for MIRANOL C2M). It should be noted that the actual concentration of the amphoteric surfactant is about half for the MIRANOL C2M solution and about a third for the MIRANOL MHT solution.

In the emulsion which did not contain any MIRANOL, the initial droplet size was about 700 nm and the emulsion was separated into two phases after 24 hours. The increase in the mean droplet size at low MIRANOL concentration was probably due to fact that there was not sufficient coverage of the amphoteric surfactant at the oil-water interface. At high concentrations, the increase in the droplet size probably resulted from a repulsion of the phospholipids from the oil-water interface.

### Test No. 4:

#### Effect of phase volume ration on mean droplet size

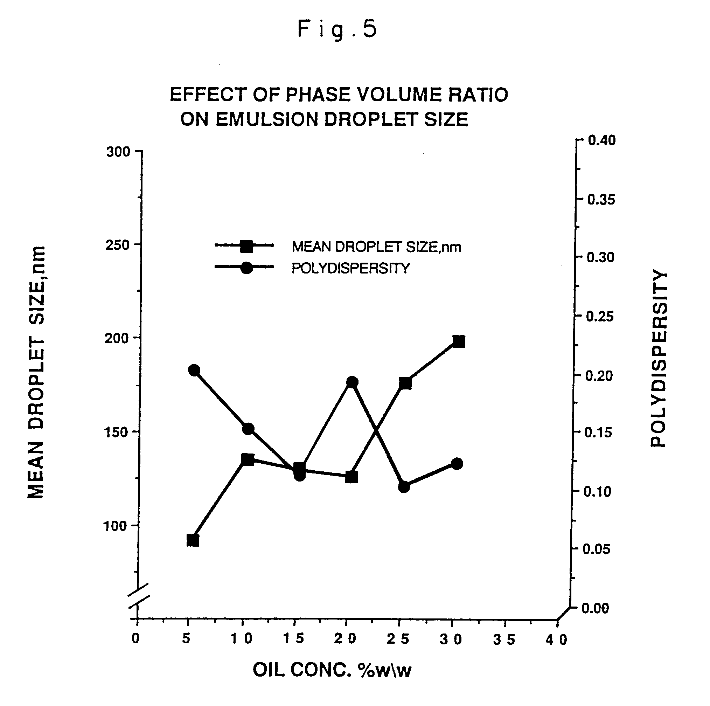

6 emulsions which differed in their oil concentration were prepared and the mean droplet size and the polydispersity for each emulsion was measured as in Test No. 2, and the results are shown in Fig. 5. As can be seen, the lowest droplet size was obtained at a concentration of oil of 5% and further, there was an increase in the mean droplet size at an oil concentration above 20%.

Test No. 5:

Effect of pH on emulsion droplet size

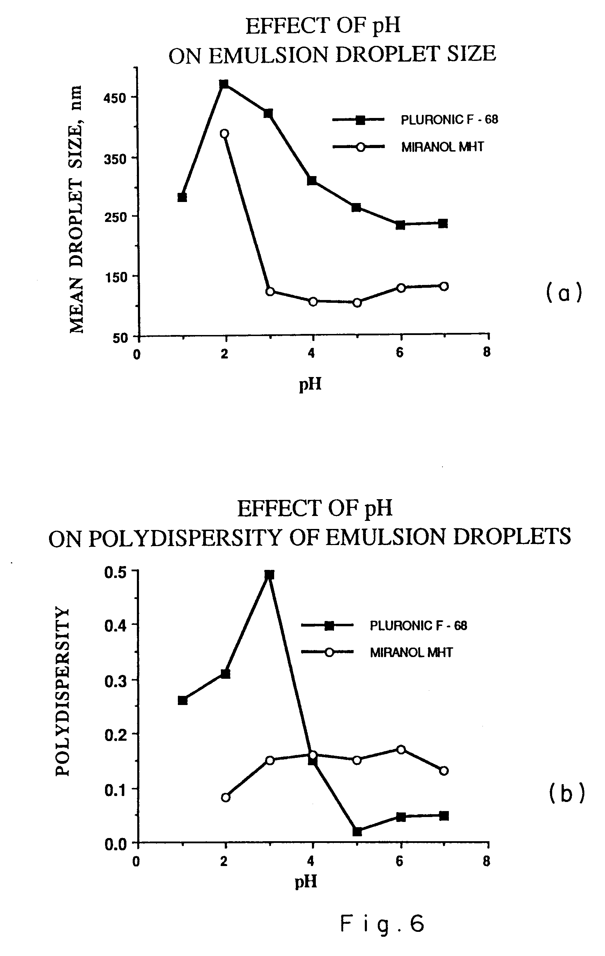

The effect of pH on the mean droplet size and the polydispersity of either Emulsion "M" or an emulsion containing the surfactant PLURONIC F-68™ (BASF, USA), was measured, and results are shown in Fig. 6. As can be seen, the mean droplet size of the emulsion of the invention (containing MIRANOL MHT) remains constant at a pH between 2-7. Against this, in the conventional emulsion (containing PLURONIC F-68) a decrease in pH causes a gradual increase in the mean droplet size. Furthermore, the MIRANOL containing emulsion, while being somewhat less homogeneous than PLURONIC F-68 containing emulsions at pH between 5-7, is considerably more homogeneous than the latter emulsion at a lower pH.

Test No. 6:

Effect of electrolyte concentration on emulsion's droplet size

The mean droplet size of emulsion containing MIRANOL MHT or PLURONIC F-68 was measured 15 min. after the addition of various divalent cation concentration.

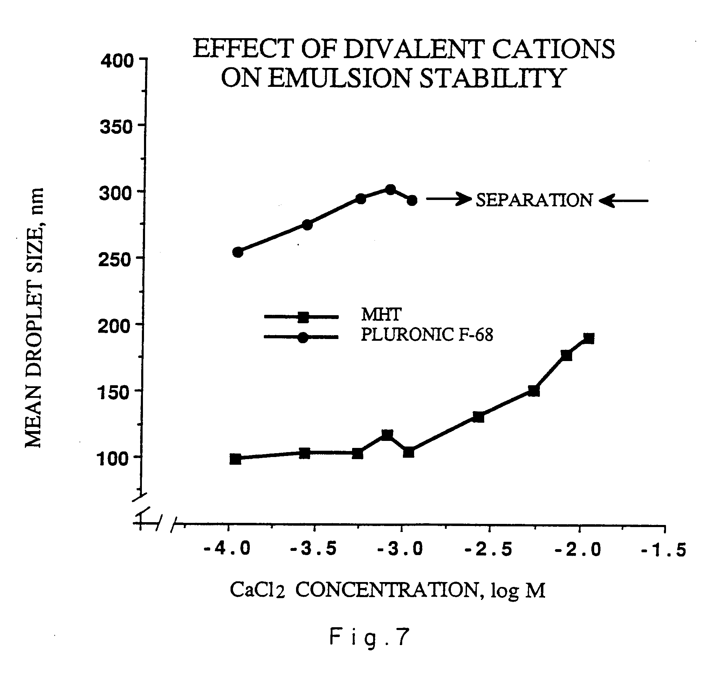

The emulsion was diluted 1:1 in an electrolyte solution. The results are shown in Fig. 7.

As can be seen the mean droplet size of the emulsion of the invention (containing MIRANOL MHT) remained constant up to an electrolyte concentration in the mM range. At higher concentrations the mean droplet size rose gradually, but the emulsions remained stable. Against this in the conventional emulsion (containing PLURONIC F-68) the rise in the electrolyte concentration caused a rapid increase in the mean droplet size and an electrolyte concentration above 25 mM caused a complete emulsion separation.

Test No. 7:

Stability of emulsions of the invention

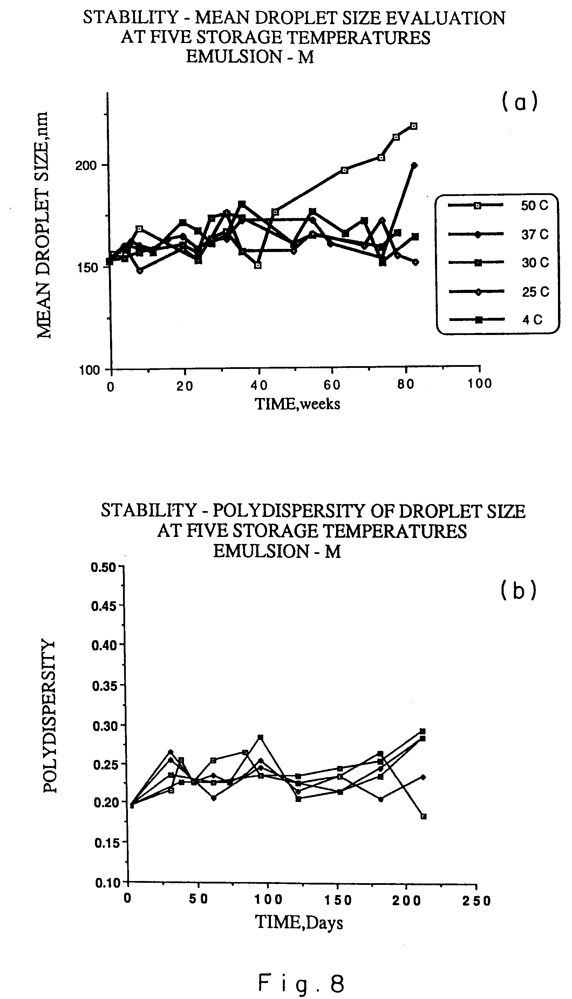

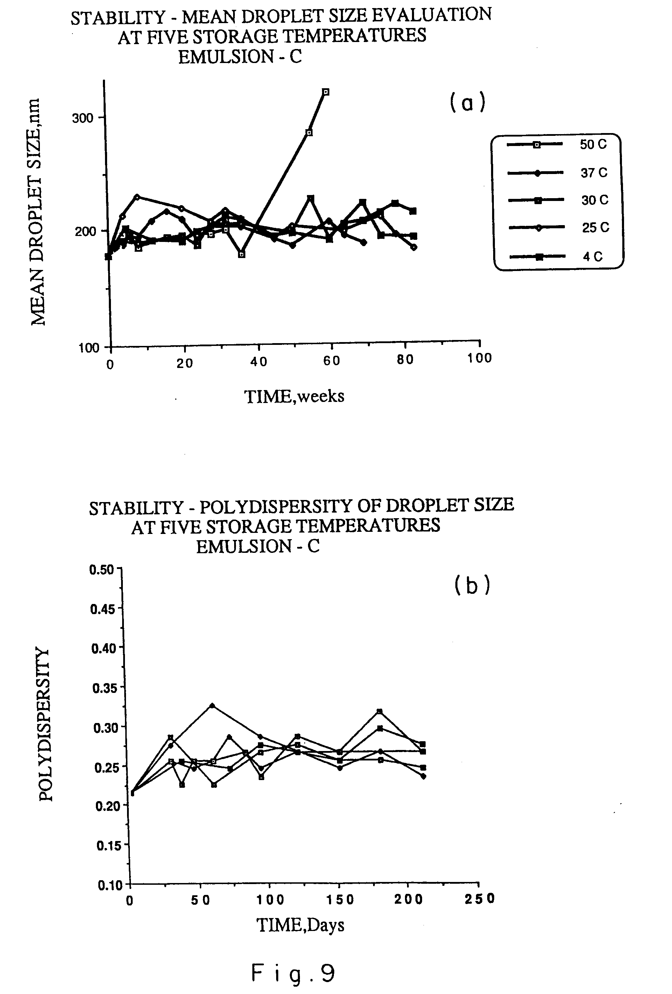

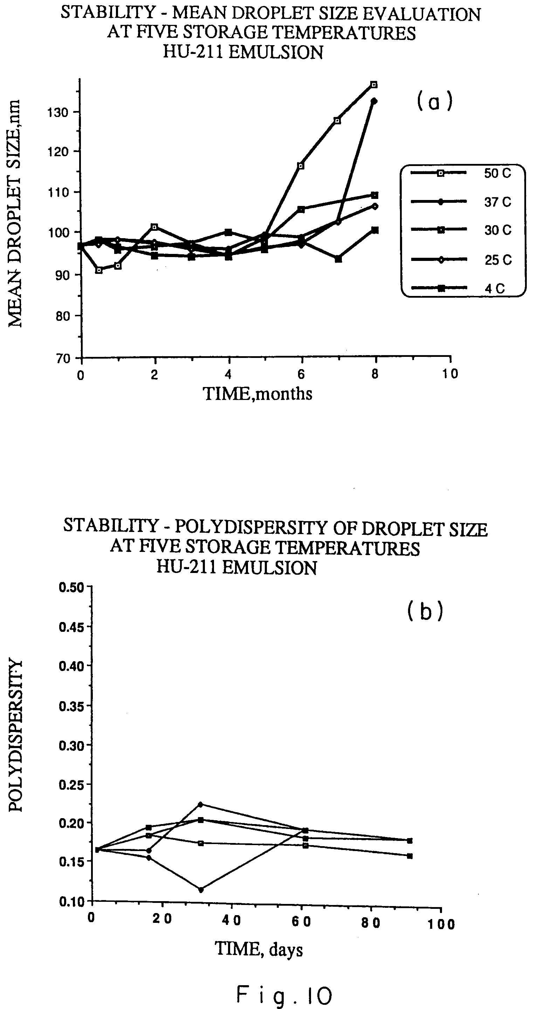

The mean droplet size and the polydispersity in Emulsion M and Emulsion C and HU-211 emulsion were measured at various storage temperatures and after various storage times and the results are shown in Fig. 8 (Emulsion M) Fig. 9 (Emulsion C) and Fig. 10 (HU-211 emulsion).

As can be seen, there was essentially no change in the physical properties of the emulsions over a tested period of 83 weeks days for Emulsion M and C when stored at 4°C, 25°C and 30°C. However, an increase in droplet size was noted at storage temperatures of 37°C and 50°C, the effect being much more pronounced at 50°C.

The physical properties of HU-211 emulsion were not affected during storage of the emulsion at 4°C, 25°C, 30°C, 37°C and 50°C over a period of at least 90 days. The mean droplet size of HU-211 emulsion stored at 50°C and 37°C started to significantly increase after 5 and 7 months of storage respectively.

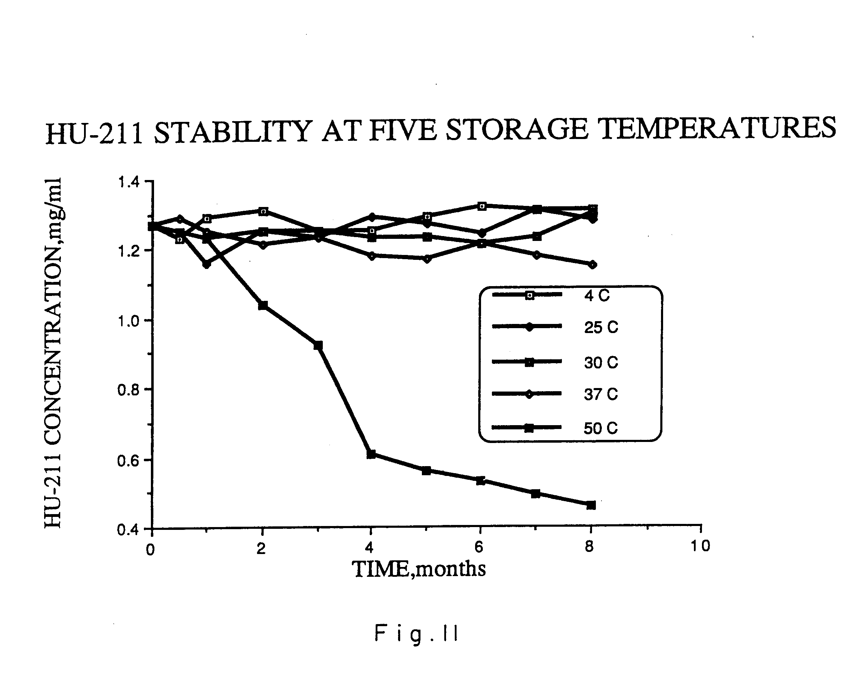

The HU-211 concentration was measured at different temperatures after various storage times and the results are shown in Fig. 11.

As can be seen, there is essentially no change in the HU-211 concentration over a period of 8 months at storage temperatures of 4°C, 25°C, 30°C and 37°C, and a marked decrease in concentration can be seen at 50°C.

Test No. 8:

Zeta-potential various storage times and temperatures

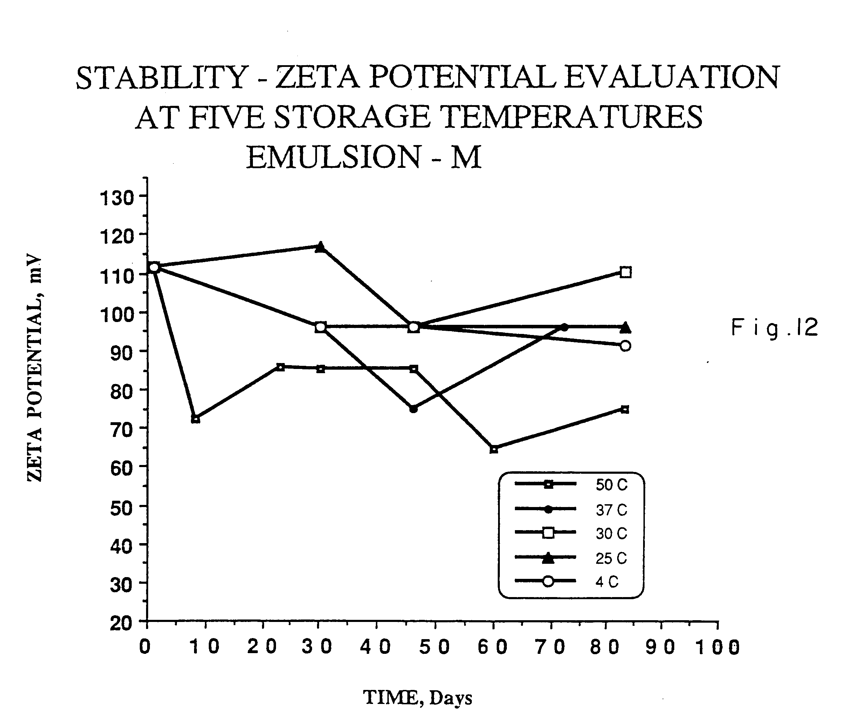

Emulsion M was stored at 5 different temperatures and the zeta-potential was measured at various storage temperatures and after various storage periods. The results are shown in Fig. 12.

In the emulsions stored at temperatures between 4 and 37°C there was only a very little decrease in the zeta-potential which is a clear sign of the emulsion's stability. Somewhat less stability, as evidenced by a decrease in the zeta potential, was observed with the emulsion stored at 50°C.

Test No. 9:

Biological Efficacy

(A) Experiments with HU-211

Albino rabbits of both sexes weighing 2.25 kg were used. All rabbits chosen had no signs of ocular pathology.

The intraocular pressure (IOP) was measured by a Digilab pneumotonometer model 30R. For IOP measurement, one drop of the local anaesthetic benoxinate HCl (Fischer Pharmaceuticals, Israel) diluted 1:3 in sterile solution was instilled into each eye. In order to ensure reproducibility of IOP readings, the animals were accustomed to the experimental procedure by repeated handling. Rabbits that showed consistent difference in the basal IOP between the eyes or between two consecutive readings in one eye, as well as any other sign of ocular irritation were excluded from the study.

Basal IOP was measured for 2 days prior to drug application by measuring the IOP 5 times over an 11 hrs. period (starting at 8am and ending at 7pm). Each time IOP was measured twice and an average of two readings was obtained.

IOP was measured at the time of drug application and at 1.5, 4, 6 and 11 hrs. thereafter.

Two groups of animals were tested, each by a different experimenter. The first group which consisted of 20 rabbits is referred to herein as " Group I" and the second group which consisted of 10 rabbits is referred to herein as "Group II". The two groups received, however, exactly the same treatment.

The treatment consisted of the application of a single dose of 50μl of a 0.1% (w/v) of HU-211 preparation in accordance with the invention into the lower cul-de-sac of the right eye of each rabbit. The eye was then kept open for a few more seconds, while excessive liquid spilling over the eyelid was wiped off. The left eye was not treated.

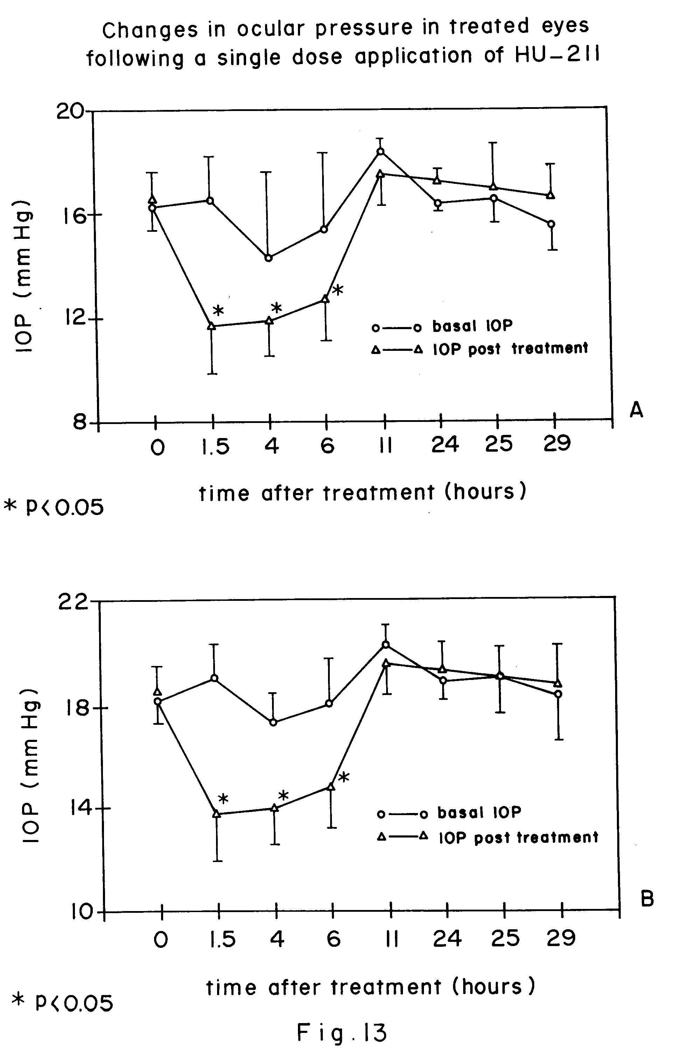

The basal IOP and that after HU-211 application is shown in Figure 13. As can be seen, following a single dose of HU-211 there was a dramatic decrease in IOP which reached a maximum level after 1½ about and which lasted for about 6 hrs. Basal IOP levels were resumed after about 11 hrs.

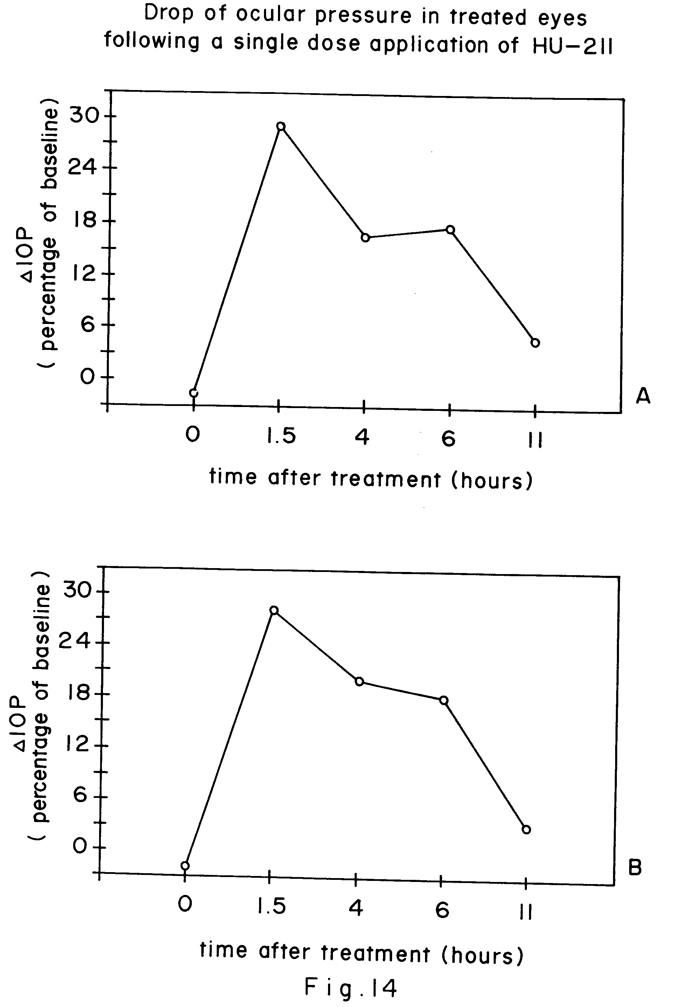

Fig. 14 shows the percentage of IOP change from the basal line level. As can be seen, the maximum decrease in IOP occurred after about 1½ hours post treatment but it persisted until about 11 hours.

In the contralateral eye a small, but significant decrease of about 11.4% and 12.1% in Group I and Group II respectively, after about 4 hours following the administration of the drug, was observed.

(B) Experiments with $\Delta^8$-THC

A composition in accordance with the invention comprising 0.4% $\Delta^8$-THC was administered to the right eyes of albino rabbits. The administration of the composition and IOP measurements were performed as described above under (A).

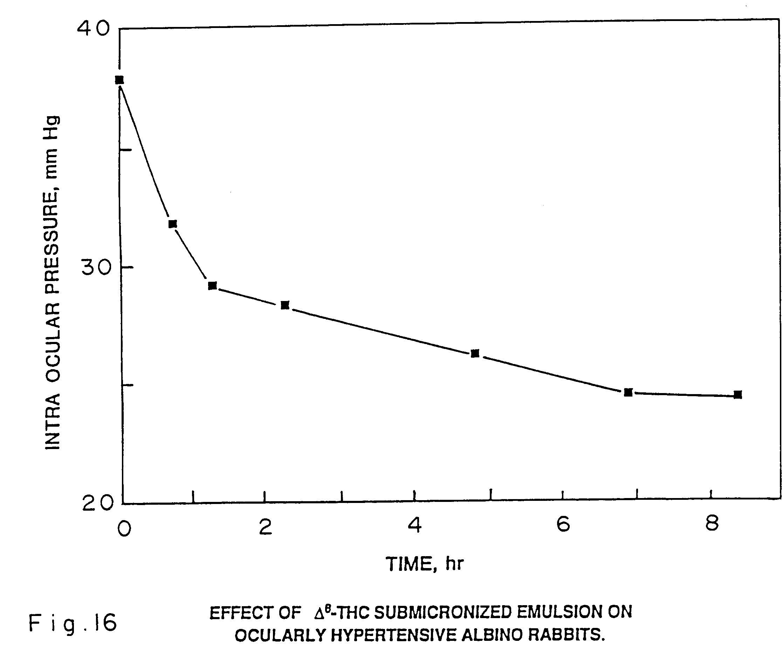

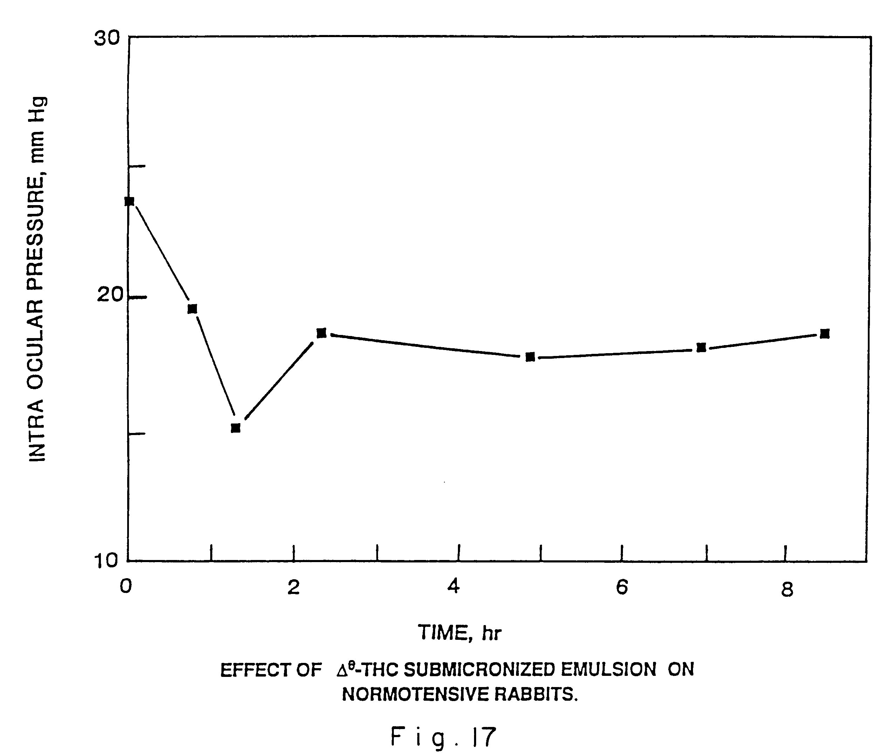

Both ocularly hypertensive and normo-tensive albino rabbits were used in the study. The results are shown in Figs. 16 and 17 and they demonstrate that $\Delta^8$-THC administered in a composition of the invention dramatically reduces the IOP in ocularly hypertensive rabbits and also in the ocularly normo-tensive rabbits. Furthermore, as can be seen the reduction of IOP is long-lasting.

Test No. 10:

Preservative Efficacy

Emulsions according to Example 1B with the drug HU-211 (0.1%) or timolol base (0.25%), were prepared. Some emulsions which did not contain a preservative served as control.

To each 20ml of emulsion, an inoculum of a microorganismal suspension (at an initial concentration of about 1-3 x $10^8$ cells per ml) was added under sterile conditions to a final concentration of about 1-3 x $10^6$ cells per ml of emulsion. Immediately upon inoculation and at 24, 48 hours, 7, 14 and 28 days thereafter, samples of 1 ml were withdrawn and eluted in a pH 7.0 sodium chloride buffer (B.P. 88 neutralizing buffer, DIFCO Laboratories, Detroit, MI, USA) to neutralize the preservatives. After about 20-30 minutes of exposure of sample to the neutralizing buffer, samples were filtered through a 0.45μm cellulose acetate filter (TE, Schueller & Schleicher). The filter and the holder were washed with 3 portions of 100 ml of sodium chloride peptone solution and then the filter was transferred to a petri dish with PCPL media which consisted of the following ingredients (per 400 ml): 20g tryptone, 1.0g yeast extract, 0.4g. dextrose, 6.0 bactoagar, 0.4g lethicin and 8.0ml Tween 80.
At 24 hours or at 2 to 5 days after the withdrawal of the sample, the number of the microorganism was counted on the petri dishes. (In order to achieve a proper count of the microorganism dilutions of the initially withdrawn sample to obtain a dilution factor of 1:10-1:50,000, were made).

The results are shown in the following Table 1:

## TABLE 1

| Organism | Preservative Emulsion Type | Number of Organisms in 1 ml of Emulsion | | | | | |
|---|---|---|---|---|---|---|---|
| | | 0 hours | 24 hours | 48 hours | 7 days | 14 days | 29 days |
| E. Coli | Blank | $3.04 \times 10^6$ | $0.18 \times 10^6$ | $0.38 \times 10^6$ | $3.63 \times 10^6$ | $10.50 \times 10^6$ | $4.75 \times 10^6$ |
| | HU-211 | $1.11 \times 10^6$ | 0 | 0 | 0 | 0 | 0 |
| | Timolol | $1.30 \times 10^6$ | 0 | 0 | 0 | 0 | 0 |
| P. aeruginosa | Blank | $7.50 \times 10^6$ | $17.20 \times 10^6$ | $20.00 \times 10^6$ | $17.50 \times 10^6$ | $20.0 \times 10^6$ | $17.55 \times 10^6$ |
| | HU-211 | $1.33 \times 10^6$ | 0 | 0 | 0 | 0 | 0 |
| | Timolol | $3.00 \times 10^6$ | 0 | 0 | 0 | 0 | 0 |
| S. aureus | Blank | $4.93 \times 10^6$ | $3.89 \times 10^6$ | $3.70 \times 10^6$ | $2.10 \times 10^6$ | $8.70 \times 10^4$ | 250 |
| | HU-211 | $4.30 \times 10^6$ | 0 | 0 | 0 | 0 | 0 |
| | Timolol | $3.10 \times 10^6$ | 150 | 0 | 0 | 0 | 0 |
| A.Niger | Blank | $5.60 \times 10^4$ | $5.00 \times 10^4$ | $1.74 \times 10^4$ | $4.00 \times 10^4$ | $0.90 \times 10^4$ | 8400 |
| | HU-211 | $4.45 \times 10^4$ | 0 | 0 | 0 | 0 | 0 |
| | Timolol | $6.00 \times 10^4$ | 0 | 0 | 0 | 0 | 0 |
| C. Albicans | Blank | $7.45 \times 10^6$ | $2.48 \times 10^6$ | $0.48 \times 10^4$ | $5.00 \times 10^4$ | $1.70 \times 10^4$ | 800 |
| | HU-211 | $1.12 \times 10^6$ | 0 | 0 | 0 | 0 | 0 |
| | Timolol | $3.05 \times 10^6$ | 0 | 0 | 0 | 0 | 0 |

The results shown in the above Table clearly indicate that the emulsion excipients did not exhibit any preservative activity at least during the first week of the experiment. however, in 3 out of the 5 microorganisms *(S. Aureus, A. niger and C. albicans)*, their viability in the control emulsion was markedly reduced 28 days after inoculation. This decrease in viability may be most likely attributed to the unfavorable living conditions which have been built up with time.

As can be seen, the combination of Thimerosal and chlorbutol was able to protect the emulsion from the contamination irrespective of the type of microorganisms.

In a different experiment similar emulsions to the above were prepared containing however one of the following preservatives instead of the combination of thimerosal and chlorbutol:

0.02% benzalkonium chloride, 0.01% Thimerosal and 0.05% chlorhexadiene gluconate (in empty or plain emulsions). Two blank emulsions without a preservative served as control for the two different neutralizing buffers. (The following two neutralizing buffers were used: NB (DIFCO special) for neutralizing thimerosal prepared according to manufacturer's recommendations (6.2g dissolved in 1,000 ml of distilled water); TAT for neutralizing benzalkonium chloride and chlorhexidine gluconate which contained (per 100 ml) - 8.0g tryptone, 2.0g lecithin and 16.0 ml Tween 20.

The results of this experiment are shown in the following Table 2:

## TABLE 2

| Organism | Preservative | Number of Organisms in 1ml Emulsion | | |
|---|---|---|---|---|
| | | Oh | 24h | 48h |
| E. coli | BZK | $2.6 \times 10^6$ | $10^7$ | $5 \times 10^6$ |
| | THI | $1.4 \times 10^6$ | 0 | 0 |
| | CHL | $5.5 \times 10^6$ | $10^7$ | $5 \times 10^6$ |
| | Con-1 | $2.6 \times 10^6$ | $10^7$ | $5 \times 10^6$ |
| | Con-2 | $1.8 \times 10^6$ | $10^7$ | $5 \times 10^6$ |
| S.aureus | BZK | $7.3 \times 10^6$ | $10^7$ | $10^7$ |
| | THI | $3.8 \times 10^6$ | 0 | 0 |
| | CHL | $3.5 \times 10^6$ | $10^7$ | $10^7$ |
| | Con-1 | $7.3 \times 10^6$ | $10^7$ | $10^7$ |
| | Con-2 | $7.3 \times 10^6$ | $10^7$ | $10^7$ |
| P. aeruginosa | BZK | $4.2 \times 10^6$ | $10^7$ | $10^7$ |
| | THI | $3.1 \times 10^6$ | 0 | 0 |
| | CHL | $4.2 \times 10^6$ | $10^7$ | $10^7$ |
| | Con-1 | $3.3 \times 10^6$ | $10^7$ | $10^7$ |
| | Con-2 | $1.3 \times 10^6$ | $10^7$ | $10^7$ |
| C. albicans | BZK | $1.0 \times 10^6$ | >600 | 433 |
| | THI | $1.3 \times 10^6$ | 130 | 0 |
| | CHL | $0.9 \times 10^6$ | >600 | 433 |
| | Con-1 | $1.6 \times 10^6$ | >600 | 379 |
| | Con-2 | $1.6 \times 10^6$ | >600 | 379 |
| A. niger | BZK | $1.5 \times 10^4$ | 29 | 43 |
| | THI | $0.5 \times 10^4$ | 0 | 0 |
| | CHL | O | 38 | 54 |
| | Con-1 | $1.5 \times 10^4$ | 46 | 43 |
| | Con-2 | $1.5 \times 10^4$ | 38 | 37 |

(*)  BZK – Benzalkonium chloride;
THI – Thimerosal;
CHL – Chlorhexidine gluconate;
Con-1 –Control with NB neutralising buffer;
Con-2 –Control with TAT neutralising buffer.

The results in the above Table clearly show that thimerosal achieves a much better preservative effect than the other tested preservatives but was not able to protect totally at 24h from C. albicans. Furthermore, the Thimerosal was not able to maintain total killing after 48 hours, since contamination was noted in some of the emulsion containing *S. aureus* and *P. aeruginosa*. The same experiment was also carried out with Chlorbutol alone instead of Thimerosal. No total preservative protection was achieved. This preservative did not meet the B.P. requirements.

**LIST OF REFERENCES**

1. Fitzgerald et al. (1987). J. Pharm. Pharmacol., 39:487-490.
2. Harmia et al. (1987). Pharm. Acta Helv., 62:322-332.
3. Saettone et al. (1988). J. Pharm., 43:67-70.
4. Meisner et al. (1989). Int. J. Pharm., 55:105-113.
5. EP-A-391369.
6. Ellis et al. (1987). J. Ocul. Pharmacol. (U.S.), 3:121-128.
7. Mechoulam, R. (ed.) (1973). Marihuana, Chemistry and Pharmacology Metabolism and Clinical Effects. Academic Press.
8. Mechoulam et al. (1987). In: Progress in Medicinal Chemistry, Vol. 24, Ellis et al. (eds.) Elsevier Science Publishers, 160-200.
9. Mechoulam et al. ( 1988). Experentia, 44:762-764.
10. Ciuchta et al. (1978). Drug. Chem. Toxico., 1:305-324.

**Claims**

1. An ophthalmic drug delivery vehicle being an oil-in-water type emulsion and comprising, by weight, about 0.5-50% oil, about 0.5-10% phospholipids and about 0.05-10% of an amphoteric surfactant.

2. A drug delivery vehicle according to Claim 1, comprising about 0.2-2% of the amphoteric surfactant.

3. A drug delivery vehicle according to Claim 2, comprising about 0.2-0.5% of the amphoteric surfactant.

4. A drug delivery vehicle according to any one of the preceding claims, wherein the amphoteric surfactant is an imidiazoline based surfactant.

5. A drug delivery vehicle according to claim 4, wherein said surfactant is MIRANOL™.

6. A drug delivery vehicle according to any one of the preceding claims wherein the ratio of phospholipids to the amphoteric surfactant is about 1:1 to 4:1 (w/w).

7. A drug delivery vehicle according to Claim 6, wherein the ratio between the phospholipids and the amphoteric surfactant is about 2:1 (w/w).

8. A drug delivery vehicle according to any one of the preceding Claims, comprising droplets of a diameter of less than about 1μm.

9. A drug delivery vehicle according to any one of the preceding claims, comprising 0.010 - 0.2 % of chlorbutol and 0.01 - 0.2 % thimerosal.

10. A pharmaceutical ophthalmic preparation comprising a drug delivery vehicle according to any one of the preceding claims and an effective amount of a hydrophobic amphophilic or lipophilic drug.

THC STABILITY
AT FIVE STORAGE TEMPERATURES
EMULSION M

THC CONCENTRATION,mg/ml

TIME, weeks

Legend:
4 C
25 C
30 C
37 C
50 C

Fig. 1

THC STABILITY
AT FIVE STORAGE TEMPERATURES
EMULSION C

THC CONCENTRATION, mg/ml

TIME, Weeks

Fig.2

50 C
37 C
30 C
25 C
4 C

Fig. 3

EP 0 521 799 A1

# Fig.4

## EFFECT OF MIRANOL CONC.
## ON EMULSION DROPLET SIZE

Fig.5

EFFECT OF PHASE VOLUME RATIO
ON EMULSION DROPLET SIZE

## EFFECT OF pH
## ON EMULSION DROPLET SIZE

(a)

## EFFECT OF pH
## ON POLYDISPERSITY OF EMULSION DROPLETS

(b)

Fig.6

Fig. 7

EFFECT OF DIVALENT CATIONS
ON EMULSION STABILITY

EP 0 521 799 A1

STABILITY - MEAN DROPLET SIZE EVALUATION
AT FIVE STORAGE TEMPERATURES
EMULSION - M

(a)

STABILITY - POLYDISPERSITY OF DROPLET SIZE
AT FIVE STORAGE TEMPERATURES
EMULSION - M

(b)

Fig. 8

21

STABILITY - MEAN DROPLET SIZE EVALUATION
AT FIVE STORAGE TEMPERATURES
EMULSION - C

(a)

STABILITY - POLYDISPERSITY OF DROPLET SIZE
AT FIVE STORAGE TEMPERATURES
EMULSION - C

(b)

F i g . 9

STABILITY - MEAN DROPLET SIZE EVALUATION
AT FIVE STORAGE TEMPERATURES
HU-211 EMULSION

( a )

STABILITY - POLYDISPERSITY OF DROPLET SIZE
AT FIVE STORAGE TEMPERATURES
HU-211 EMULSION

( b )

F i g . 10

HU-211 STABILITY AT FIVE STORAGE TEMPERATURES

Fig. 11

## STABILITY - ZETA POTENTIAL EVALUATION
## AT FIVE STORAGE TEMPERATURES
## EMULSION - M

Fig.12

Changes in ocular pressure in treated eyes
following a single dose application of HU–2II

Fig. I3

Drop of ocular pressure in treated eyes
following a single dose application of HU-211

Fig.14

Fig. 15

Changes in ocular pressure in contralateral eyes following a single dose application of HU-211

* P<0.05

Fig. 16

INTRA OCULAR PRESSURE, mm Hg

TIME, hr

EFFECT OF Δ⁸-THC SUBMICRONIZED EMULSION ON
OCULARLY HYPERTENSIVE ALBINO RABBITS.

INTRA OCULAR PRESSURE, mm Hg

TIME, hr

EFFECT OF Δ⁸-THC SUBMICRONIZED EMULSION ON NORMOTENSIVE RABBITS.

Fig. 17

**European Patent Office**

# EUROPEAN SEARCH REPORT

Application Number

EP 92 40 1927

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | EP-A-0 391 369 (YISSUM RESEARCH DEVELOPM. COMP. OF THE HEBREW UNIVERS. OF JERUSALEM) * the whole document * * claim 6 * | 1-10 | A61K9/107 |
| Y | EP-A-0 253 472 (THE GREEN CROSS CORPORATION ET AL.) * the whole document * | 1-10 | |
| Y | DE-A-3 020 129 (DR. THILO & CO GMBH) * claims 1,4 * * page 9; examples 5-7 * | 1-10 | |
| Y | FR-A-2 276 832 (SANDOZ S.A.) * the whole document * | 1-10 | |
| A | US-A-4 146 499 (ROSANO) *the abstract; column 7, lines 8-38* | 1-10 | |
| | | | **TECHNICAL FIELDS SEARCHED (Int. Cl.5)** |
| | | | A61K |

The present search report has been drawn up for all claims

| Place of search | Date of completion of the search | Examiner |
|---|---|---|
| THE HAGUE | 24 SEPTEMBER 1992 | BENZ K.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

\& : member of the same patent family, corresponding document

EPO FORM 1503 03.82 (P0401)